# EUROPEAN PATENT SPECIFICATION

(11) **EP 3 386 453 B1**
(45) Date of publication and mention of the grant of the patent: **01.04.2020**
(21) Application number: 15810773.0
(22) Date of filing: 11.12.2015
(51) Int. Cl.: A61F 13/00

(54) **A WOUND DRESSING LAMINATE, METHODS OF MANUFACTURING THE WOUND DRESSING LAMINATE AND USES THEREOF**
WUNDVERBANDLAMINAT, VERFAHREN ZUR HERSTELLUNG DES WUNDVERBANDLAMINATS UND VERWENDUNGEN DAVON
STRATIFIÉ DE PANSEMENT POUR PLAIE, PROCÉDÉS DE FABRICATION DU STRATIFIÉ DE PANSEMENT POUR PLAIE ET LEURS UTILISATIONS

(43) Date of publication of application: 17.10.2018
(73) Proprietor: Pharmaplast SAE, Alexandria 23512 (EG)
(72) Inventor: ATTEIA, Mamdouh, Alexandria 23512 (EG); GAD, Wassim, Alexandria 23512 (EG); AZIZ, Dina, Alexandria 23512 (EG); ZAKI, Mena, Alexandria 23512 (EG)
(74) Representative: Holme Patent A/S
(86) International application number: PCT/IB2015/059542
(87) International publication number: WO 2017/098310

(56) References cited:
- WO-A1-99/67456
- WO-A1-2015/081769

## Description

The present invention relates to a body-shape wound dressing comprising a wound dressing laminate, which wound dressing laminate comprises a wound-facing non-woven layer composed of non-thermoplastic fibres combined into a coherent laminate structure with a non-woven backing layer composed of thermoplastic fibres, and body-shape wound dressings for exuding wounds made of this wound dressing laminate.

Exudation from many types of skin lesions is normal during the healing process. Included are such wounds as Stage II and Stage III ulcers, second and third degree burns, skin grafts and donor sites, deep dermal abrasions and lacerations. Conventional bandages or wound dressings readily absorb fluids, when used for such lesions, but become saturated with exudate seeping from the open wound, necessitating frequent bandage or dressing changes. The frequent changes cause irritation of the wound, discomfort to the patient and increased health care costs.

Smith & Nephew, Inc. manufactures the RAPID RHINO™ Epistaxis Products, which is an inflatable tamponade for epitaxis (nose bleeding). A PVC balloon is covered by CMC (Carboxymethyl cellulose) infused knitted fabric reinforced by nylon. When the device is soaked in water a self-lubricating gel is formed which eases insertion into the nose. The balloon is inflated until the tamponade conforms to the nasal anatomy whereby the tamponade keeps *in situ* until bleeding stops. The tamponade is removed about 24-72 hours after treatment. Thus structure is complicated to make, and the knitted surface is not pleasant in contact with painful exuding wounds.

Smith & Nephew, Inc. also manufactures strong, absorbent, gelling, non-woven fibre dressings, named DURAFIBRE, which are effective in exudate management. The DURAFIBRE dressing is composed of 20% natural strengthening cellulose fibres and 80% gelling cellulose ethyl sulphonate fibres (CES) made into a spun matrix. This matrix transforms into a soft cohesive gel as it is hydrated with exudate and it conforms intimately to the wound bed in close contact with the wound surface without adhering, thereby keeping a moist wound environment that promotes healing, reduces the risk of exudates pooling; reduces the potential for dead space between the wound and dressing interface and minimizes the potential for trauma on dressing removal. DURAFIBRE has a somewhat high absorbent capacity and can for little and medium exuding wound stay in place for up to 7 days.

However, for highly exuding wounds DURAFIBRE needs to be changed frequently, which in itself can be painful. Even though DURAFIBRE tends not to stick to the wound the mere manipulation and contact cause heavy discomfort to the patient. A dressing should be changed based on clinical indications, e.g. if excessive exudate is locked and the dressing begins to leak. However every time a dressing is changed it results in exposure of the wound to the non-sterile environment and increased risk of contamination.

A wound dressing, which would have even better capability of absorbing exudate and improved ability to transmit absorbed moisture at a desired rate through the dressing to the distal surface of the dressing, thus the surface furthest removed from the wound when the dressing is in place over the wound, where it can evaporate from the dressing to reduce excessive moisture accumulation, would have many advantages. Such a wound dressing could remain in place over the wound without needing to be changed for a longer period of time than conventional wound dressings, and the wound would not be disturbed as frequently as a result of dressing changes, and the patient would not experience as much discomfort from the associated trauma, and risk of infection is reduced.

To that aspect international patent application no. WO2010/38231 discloses a wound dressing composed of polyurethane foam coated on the inner surfaces with hyaluronic acid, or a wound dressing composed of sodium carboxymethyl cellulose and ionic silver.

US patent no. 4,977,892 suggests another wound dressing comprised of a wound dressing laminate having a hydrophilic absorbent polymeric layer, e.g. of sodium carboxymethyl cellulose preferably dispersed in styrene-butadiene-styrene block copolymer. The hydrophilic absorbent polymeric layer is attached to a fabric layer. The hydrophilic absorbent polymeric layer is applied to the fabric layer, which fabric layer is made of spun-bonded staple fibres. A monomer solution of the polymer is poured onto the fabric layer and is thereafter cured to yield the polymeric layer. A pressure-sensitive polyurethane adhesive serves to maintain contact between polymeric film and the facing fabric layer.

International patent application no. WO94/16746 describes a wound dressing having a wound-contacting surface that comprises carboxymethyl cellulose filaments capable of absorbing at least 15 times their own weight of 0.9% by weight aqueous saline solution to form a swollen transparent gel. The dressing, when thus swollen to form a transparent gel, retains sufficient fibrous character to be removed as a coherent dressing from a wound. The carboxymethyl cellulose filaments can be used to treat a traumatic surgical or chronic wound.

Methods of manufacturing carboxymethyl cellulose fibres and filaments are well known to persons skilled in the art. An exemplary method is described in the Master's Thesis in the International Masters program Chemical Engineering "Absorbent cellulose based fibres, Investigation of carboxylation and sulfonation of cellulose", by Magnus Bergh, Department of Chemical and Biological Engineering, Division of Materials Science, CHALMERS UNIVERSITY OF TECHNOLOGY, Göteborg, Sweden, 2011. Another reference is *"*Synthesis and characterization of carboxymethyl celluloses (CMC) from non-wood fibres I. Accessibility of cellulose fibres and CMC synthesis" by Claudia Barba1, Daniel Montané1, Marguerite Rinaudo and Xavier Farriol1, in Cellulose 9: 319-326, 2002, Kluwer Academic Publishers.

From International patent application no. WO99/67456 is known a cavity wound dressings mad of a fabric that comprises first and second webs of gel-forming fibre needled to the first and second sides respectively of a textile fibre scrim.

It is a main aspect of the present invention to provide an alternative material for making dressings for exuding wounds.

It is a further aspect of the present invention to provide dressings made of the material.

It is yet an aspect of the present invention to provide a wound dressing for exuding wounds having a lengthening of the useful life of the wound dressing and minimizing macerations.

It is yet an aspect of the present invention to provide a high-absorbency and high-strength wound dressing.

It is yet an aspect of the present invention to provide a wound dressing for large-area exuding wounds.

It is yet an aspect of the present invention to provide a wound dressing for exuding wounds that has a high ability to conform to body-shape and wound shape.

It is yet an aspect of the present invention to provide a wound dressing for exuding wounds having better tear resistance than conventional wound dressings for exuding wounds, even when retaining a high content of exudate.

The novel and unique whereby these and other aspects are achieved consist in that
- the non-thermoplastic fibres of the wound-facing non-woven layer are carboxymethyl cellulose fibres,
- the thermoplastic fibres of the non-woven backing layer are selected from the group of fibres comprising polyurethane fibres, hydrophobic polypropylene fibres and/or spunlaced fibres, wherein
- the wound-facing non-woven layer and the non-woven backing layer have been laminated together by needle-punching.

Needle-punching of non-woven fibre layers creates mechanically orienting and interlocking of the fibres of non-woven webs. The needle punching technique is well known in the art of making non-woven products and will not be discussed in details in the present application. The mechanical interlocking is achieved with a plurality of barbed felting needles of a needle loom repeatedly passing into and out of the fibres of the web(s) to provide coherency between fibres and make the coherent structure strong and tear resistant.

Combining the first non-woven non-thermoplastic fibre layer with the second non-woven layer of thermoplastic fibres is achieved without thermal application or binders. Thus the wound dressing laminate preserves the binding property of the thermoplastic fibres. Use of thermal application or additional binders to make the wound dressing laminate coherent would render the wound dressing laminate rather stiff, negatively affect the absorbing properties of the carboxymethyl cellulose fibres, and deprive the polyurethane fibres, hydrophobic polypropylene fibres and/or spunlaced fibres of binding properties. Entangling the fibres of the layer of thermoplastic polyurethane fibres, hydrophobic polypropylene fibres and/or spunlaced fibres with the fibres of the carboxymethyl cellulose fibre layer by needle-punching makes the resulting wound dressing laminate strong with superior resistance to tear and delaminating. The carboxymethyl cellulose fibres of the wound facing layer absorbs exudates and gradually gels. When a wound dressing is made of the wound dressing laminate of the present invention the backing layer advantageously keeps the wound dressing laminate as an integral structure that can be taken from the wound without deterioration and disintegration of the wound dressing laminate. Moreover, the provision of the thermoplastic fibres in the wound dressing laminate makes it possible to combine two wound dressing laminates of the present invention without stitching or sewing said wound dressing laminates together. This way a joint between opposite wound dressing laminates can be made as comfortable to the patient as possible due to minimal protruding seams. This way the exuding wound is the least possible subjected to protruding and annoying parts of a body-shape wound dressing that might be invasive on the already vulnerable exuding wound and adjacent sensitive skin.

The body-shape wound dressing made of the soft and pliable wound dressing laminate conforms easily to the relevant body-shape. When the body part is moved, turned, stretched or relaxed no noticeable impact is caused to the body-shape wound dressing, even if the body part is very curved. The wound dressing laminate of the inventions reconfigures and adapts in response to any movements and body-part curvature and shape. Body-shaped dressings will be discussed in further details below.

Even when high amounts of exudate are absorbed by the inventive wound dressing laminate the different fibres keep together, and the wound dressing laminate maintains soft and smooth against the exuding wound and surrounding sensitive skin without sticking to said wound.

The wound-facing non-woven carboxymethyl cellulose fibre layer may simply be provided as a loose airlaid layer on top of which the polyurethane fibres, hydrophobic polypropylene fibres and/or spunlaced fibres of the non-woven backing layer are similarly airlaid loosely. Subsequently, needle-punching is conducted from one or both sides of the stacked layers, e.g. from the side of the non-woven backing layer to create a wound dressing laminate featuring a side being mainly non-thermoplastic and an opposite side being mainly thermoplastic.

In the alternative the carboxymethyl cellulose fibres of the wound-facing non-woven layer can be made into a coherent felt before the polyurethane fibres, hydrophobic polypropylene fibres and/or spunlaced fibres of the non-woven backing layer are airlaid on top of said felt. The fibres of the non-woven backing layer are then partly integrated into the felt and with each other by needle-punching to create the wound dressing laminate with the non-thermoplastic side and the thermoplastic side. The felt can e.g. be made in a separate first process by needle-punching, stored on bobbins, and rewound continuously as a base felt for the fibres of the non-woven backing layer.

Yet an alternative is air-laying the carboxymethyl cellulose fibre of the wound-facing non-woven layer at a first air-laying station, needle-punching at a first needle-punching station to obtain the felt, then air-laying the polyurethane fibres, hydrophobic polypropylene fibres and/or spunlaced fibres of the non-woven backing layer on top of the felt at a second air-laying station, and finally needle-punching the felt and the non-woven backing layer into the coherent wound dressing laminate at a second needle-punching station.

Yet an alternative is simply to manufacture both the wound-facing non-woven layer and the non-woven backing layer at separate stations and combine those by needle-punching to obtain the wound dressing laminate.

In a preferred embodiment the weight of the wound-facing non-woven layer is between 50 g/m² and 250 g/m² before needle-punching. The weight of the wound-facing non-woven layer reflects the capacity of the wound dressing laminate to absorb and retain exudates.

The carboxymethyl cellulose fibres may comprise sodium carboxymethyl cellulose fibres, which are inexpensive to manufacture and are commercial available.

A preferred embodiment of a backing layer is composed of thermoplastic polyurethane fibers. In an alternative embodiment the backing layer is composed entirely or has a majority of spunlaced polyester fibres, or is made of or include hydrophobic polypropylene fibres.

The wound dressing laminate of the present invention is manufactured without additional use of adhesives for coherency between any of the fibres. Thus the needle-punching may be the sole means to obtain the coherency between the two non-woven fibre layers and no undesired bonding chemicals are at risk of being transferred to the wound bed from a wound dressing structure bonded by conventional binders, such as adhesives for making wound dressing laminates. Further, the risk of delaminating is substantially reduced by using fibre entangling by needle-punching.

On the other hand amongst chemicals that might have beneficial effects in a wound dressing made of the wound dressing laminate an antimicrobial agent can be mentioned. Such an antimicrobial agent can advantageously be included in the wound-facing non-woven layer by incorporation in the carboxymethyl cellulose fibres at the carboxymethyl cellulose fibres manufacturing stage. The antimicrobial agent can e.g. include one or more of ionic silver, silver aluminosilicate, glass silver, such as e.g. the inorganic silver antimicrobial product IONPURE obtainable from ISHIZUKA GLASS CO.,ltd. 1880, Kawai-cho, Iwakura-shi, Aichi 482-8510 Japan; or nano-silver in the form of nanoparticles of silver and/or silver oxide of sizes between 1 nm and 100 nm.

The majority of fibres exposed at the non-thermoplastic felt side of the wound dressing laminate, the wound-facing side, are carboxymethyl cellulose fibres. The majority of fibres exposed at the thermoplastic layer side, the backing side, are thermoplastic polyurethane fibres, hydrophobic polypropylene fibres and/or spunlaced fibres. This way the completed wound dressing laminate gets both a non-thermoplastic side and a thermoplastic side, and said thermoplastic component or side can be utilized to bind several pieces of corresponding wound dressing laminate together.

The present invention further relates to a method of manufacturing the body-shape wound dressing described above.

The method comprises the steps of
- providing a non-woven fibre layer composed of non-thermoplastic carboxymethyl cellulose fibres,
- providing, on top of the non-woven fibre layer composed of non-thermoplastic carboxymethyl cellulose fibres, a non-woven fibre layer composed of thermoplastic fibres selected from the group of fibres comprising polyurethane fibres, hydrophobic polypropylene fibres and/or spunlaced fibres,
- combining the non-woven fibre layer composed of non-thermoplastic carboxymethyl cellulose fibres and the non-woven fibre layer composed of thermoplastic fibres selected from the group of fibres comprising polyurethane fibres, hydrophobic polypropylene fibres and/or spunlaced fibres into a coherent laminate structure by needle-punching, and either
- cutting the pieces for the body-shape wound dressing from the wound dressing laminat, and welding together the pieces at least partly along opposite cut edges of said pieces thereby creating selected openings for application of the body-shape wound dressing on a body part, or
- placing two wound dressing laminates on top of each other, welding the two wound dressing laminates to each other along a welding seam pattern defining a selected body-shape wound dressing, with or without one or more openings for application of the body-shape wound dressing on a body part, and separating the welded body-shape wound dressing from the opposite two wound dressing laminates by cutting.

The present invention further relates to a method of manufacturing the wound dressing laminate described above for use in a body-shape wound dressing described above.

The method comprises the steps of
- air-laying a non-woven fibre web composed of non-thermoplastic carboxymethyl cellulose fibres,
- air-laying a non-woven fibre web composed of thermoplastic fibres selected from the group of fibres comprising polyurethane fibres, hydrophobic polypropylene fibres and/or spunlaced fibres on top of the non-woven fibre web composed of non-thermoplastic carboxymethyl cellulose fibres, and
- combining the non-woven fibre web composed of non-thermoplastic carboxymethyl cellulose fibres and the non-woven fibre web composed of non-thermoplastic fibres selected from the group of fibres comprising polyurethane fibres, hydrophobic polypropylene fibres and/or spunlaced fibres into a coherent laminate structure by needle-punching.

The needle-punching can be done from any of the wound dressing laminate sides, however for the first embodiment in particular the needle-punching may preferably be done from the non-woven backing layer side. For the second embodiment of the method of the present invention the needle-punching can be done from any side. What is important is that the needle-punching is done to an extent wherein the fibres are coherent so that the wound dressing laminate becomes strong, with high tear resistance and little risk of tendency to delaminating. Thus needle-punching can also be done from both sides.

The present invention utilizes a wound dressing manufactured of the wound dressing laminate described above.

The wound dressing laminate is particular suited for manufacturing wound dressings shaped to conform to a body-shape of a human, in particular wearable dressings that can surround and cover a wounded body part.

To that aspect the wound dressing can be composed of at least two opposite wound dressing laminate layers that are welded together along at least a part of adjacent edges, however more than one piece of wound dressing laminate can be joined to design substantially any imaginable shape.

The opposite wound dressing laminate layers can preferably be welded together by ultrasonic welding. E.g. the pieces to be joined are put in contact, and ultrasound is applied at the welding seam locations intended for joining.

Thus, due to the inclusion of the thermoplastic fibres ultrasonic welding suddenly becomes possible. The ultrasonic welding is fast and creates a uniform and smooth welding seam that can hardly be felt by the patient wearing the wound dressing of the present invention. Moreover, the present invention devises a means to combine, without thermal application, two layers of coherent pliable soft structured wound dressing laminate that has high ability to absorb and retain exudates. Emphasis is made that the two wound dressing laminate layers is not limited to being welded together in overlapping relationship by ultrasonic welding, they can also be fused together by ultrasonic welding along opposite free contacting edges.

The ultrasonic welding process induces vibrations that cause local melting of the thermoplastic plastic fibres along the welding seam due to absorption of vibration energy. Ultrasonic welding is much faster than conventional stitching and sewing together of pieces of wound dressing for body-shape wound dressings, as well as being fast, accurate, repeatably and inexpensive. The area melted by application of the highfrequency ultrasonic acoustic vibrations dries and cures at considerably shorter time than adhesives. Similarly stitching wound dressing laminate layers together are extremely time-consuming. A further advantageous aspect is that the ultrasonic welding can be automated and modified to make any kind of body-shape dressing with clean and precise welding seams as joints. Manual interactions in the rapid manufacturing process are very limited and considerable manufacturing costs are saved.

Examples of body-shape wound dressing of the present invention include but are not limited to a three-dimensional wound dressing configured as a tube, a glove, a T-shirt, a finger wrap, a glove, a mitt, a sleeve, a vest, a jacket, a mask, a skull cap, a neck tube, a girdle, shorts, pants, leggings, a leg wrap, a sock or a toe wrap.

The non-thermoplastic felt side of the wound dressing laminate is expediently used as the wound contacting side or face due to the ability to absorb and retain high content of exudate conferred by the gelling property of carboxymethyl cellulose fibres.

For the frequency of change of the body-shape wound dressing according to the present invention, if it is applied to heavily exuding or sloughy exuding wounds, daily initially replacing may be needed, but as healing progresses and the amount of exudate decreases, the interval between changes may be extended to up to 7 days in some circumstances. So the improved tear property in combination with the carboxymethyl cellulose fibres ability to gel, thus to absorb and retain large amounts of exudate, makes the wound dressing laminate of the present invention a highly attractive alternative to existing wound dressings.

Irrespective of which method is used for realizing a three-dimensional body-shape wound dressing a suitable cutting step is die-cutting.

The optimum welding step is performed by ultrasonic welding as described above.

The thickness of the wound-facing non-woven layer composed of non-thermoplastic fibres and of the non-woven backing layer composed of thermoplastic fibres before being combined into a coherent wound dressing laminate structure may be selected differently with the view of tailoring wound dressings to different wound dressing tasks. The thickness of the wound dressing laminate may be substantially the same as the sum of the individual felt and backing layer, or smaller.

The structure and use of an exemplary wound dressing laminate are illustrated in the accompanying drawing wherein
fig. 1 is a body-shape wound dressing shaped as a glove and made of the wound dressing laminate according to the present invention,
fig. 2 is a sectional enlarged view taken along line II-II in fig. 1,
fig. 3 is an enlarged fragmentary view of the encased detail indicated in fig. 1 of the fibre structure of the glove seen in fig. 1, wherein the fragment is taken in the vicinity of an ultrasonic welding seam of the two opposite layers of the glove,
fig. 4 is a principle diagram of an exemplary production line for manufacturing the wound dressing laminate according to the present invention, and
fig. 5 is a principle diagram of a schematically shown production line for a body-shape wound dressing manufactured of the wound dressing laminate according to the present invention.

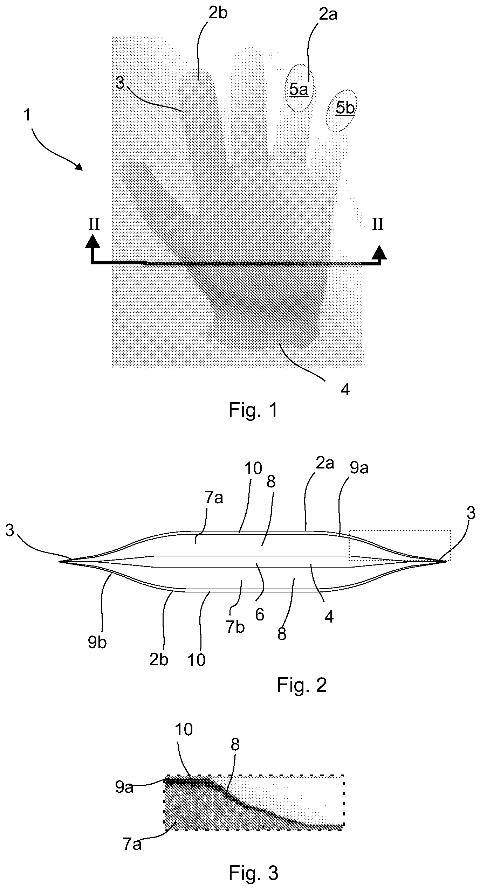

The body-shaped wound dressing seen in fig. 1 is a glove 1 composed of two pieces of oppositely arranged hand-shaped wound dressing laminates 2a,2b, which have been ultrasonically welded together along welding seam 3 to create the glove 1 having an access opening 4 for introduction of the corresponding body part, which in the present case is a hand due to the exemplary glove-shape. To illustrate the versatility of the body-shape wound dressing according to the present invention, the tip of two fingers of the glove 1 is cut off leaving exit openings 5a,5b, in the present example for exiting two finger tips. Irrespective of the specific body-shape dressing further individualization is possible to serve the patient's need for customized wound coverage and wound compliance. Such customization could be in form of slits, through-openings through the thickness of the wound dressing laminate, and larger cut-outs, all of which can be arbitrarily placed and sized.

Fig. 2 shows a principle, sectional view along line II - II in fig. 1. The adjacent pieces of wound dressing laminate 2a,2b are ultrasonically welded to each other across their adjacent contacting edges by means of welding seam 3, thereby delimiting a compartment 6 for accommodating the intended wounded body part, a human hand in the present case, which is inserted into the compartment 6 via the access opening 5.

The wound dressing laminates 2a,2b are both composed of a non-woven layer 7a,7b of non-thermoplastic carboxymethyl cellulose fibres 8. These opposite non-woven layers 7a,7b define the walls of the compartment 6 and face the wounded areas of the patient's skin.

The non-woven layers 7a,7b of non-thermoplastic carboxymethyl cellulose fibres 8 are laminated by needle-punching to a non-woven backing layer 9a,9b composed of thermoplastic fibres 10 selected from the group of fibres comprising polyurethane fibres, hydrophobic polypropylene fibres and/or spunlaced fibres to create the coherent laminate structure 2a,2b used in the present invention. The wound-facing non-woven layers 7a,7b are thicker than the non-woven backing layer 9a,9b and able to absorb and retain substantial amounts of exudate during wound healing while the non-woven backing layer 9a,9b serves to maintain the integrity of the wound dressing 1 when it inevitably changes along with the continuously exudate absorption. Due to the fibre entangling obtained by the needle-punching the non-woven backing layer 9a,9b is able to preserve the integrity and coherency of the various fibres and wound dressing laminate layers to such a high degree that the wound dressing made of the wound dressing laminate of the present invention can be removed and/or replaced without deteriorating and disintegrating, which makes said procedure much more gentle than with known wound dressings that tend to disintegrate as a consequence of the increasing content of absorbed exudate. The absorbing carboxymethyl cellulose fibres 8a,8b has the great advantage of having a low tendency to stick to the wound bed.

The fibre entangling is shown fig. 3, which is an enlarged scale view of the dotted box seen in fig. 2 encasing a fragment of the wound dressing laminate 2a, where the view of fig. 3 depictures a part of the welding seam 3.

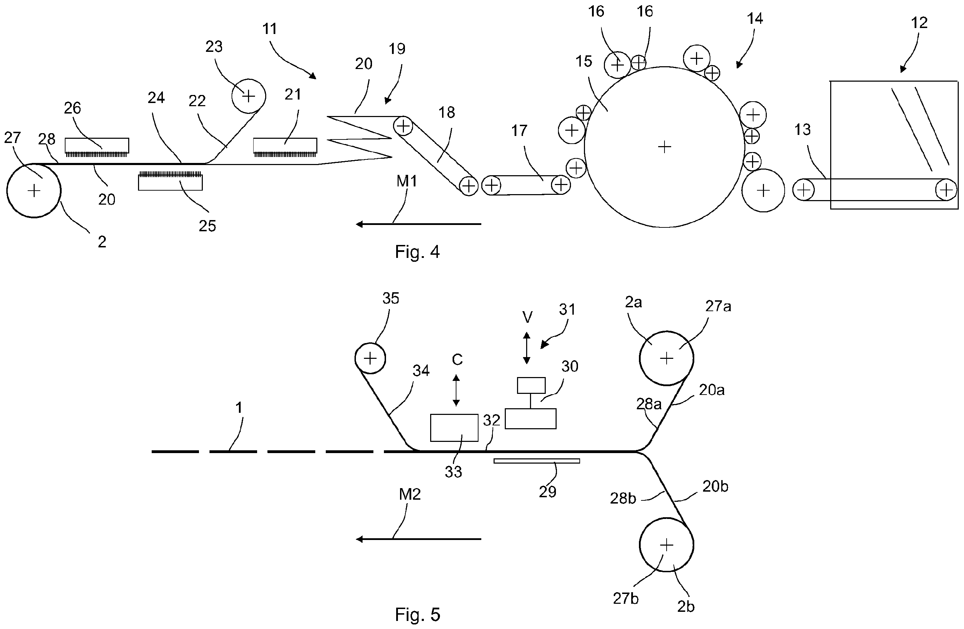

An exemplary production line 11 for the wound dressing laminate is shown in fig. 4. The machine direction is indicated by Arrow M1, thus the production line starts to the right in fig. 4.

The production line 11 comprises an opener 12 wherein the bulk of a carboxymethyl cellulose fibre tow can be increased by separating the filaments and deregistering the crimp of the tow (the tow is not shown at the beginning of the production line). The opened carboxymethyl cellulose fibre tow can then pass on a first conveyor belt 13 to a carding station 14. At the carding station 14 the carboxymethyl cellulose fibre of the carboxymethyl cellulose fibre tow can be brought into a sliver form or into a flat non-woven web, as well as impurities can be removed. The center drum 15 of the carding station 14 is attacked by a plurality of smaller rollers 16 the surfaces of which are covered with many teeth or wires to create the sliver web form or non-women web form.

The carded carboxymethyl cellulose fibre web (not shown) proceeds on a second conveyor belt 17, further onto a third conveyor belt 18 for delivery into a cross-lapping station 19, where the carded carboxymethyl cellulose fibre web is folded a number of times for adding thickness to create a suitable thick felt 20.

The felt 20 of carboxymethyl cellulose fibre is then needle punched at a first needle loom 21. A premade non-woven web 22 of thermoplastic fibres is supplied from a first bobbin 23 onto the felt 20, thereby creating a loose pre-laminate structure 24, which is needle-punched at a second needle loom 25 from one side and subsequently needle-punched at a second needle loom 26 from the opposite side to finally obtain the coherent wound dressing laminate 2, which is wound on a second bobbin 27 for later use in making wound dressings of various kinds. The non-woven web 22 of thermoplastic fibres is entangled together with the felt 20 so that said non-woven web 22 eventually becomes coherent with the felt 20 and becomes the backing layer 28 that contributes to the high degree of tear resistance.

Fig. 5 is a principle diagram of a production line for a schematically shown body-shape wound dressing manufactured of the wound dressing laminate, e.g. manufactured using the production line 11 shown in fig. 4 to obtain the glove 1 seen in fig. 1. The machine direction is indicated by Arrow M2.

Two layers of wound dressing laminate 2a,2b is wound from two second bobbins 27a,27b so that opposite respective carboxymethyl cellulose fibre felts 20a,20b converge into contact and can pass between a base plate 29 and a sonotrode 30 of an ultrasonic welding station 31. The respective thermoplastic backing layers 28a,28b of the contacting two layers of wound dressing laminate 2a,2b are free and exposed. Application of the sonotrode 30 to the two layers of wound dressing laminate 2a,2b creates welded areas, seams and/or zones, including the desired welding seam 3 of the glove 1. The thermoplastic property of a backing layer 28 absorbs the vibratory energy induced to the stack of the two layers of wound dressing laminate 2a,2b by the vibration provided by the sonotrode 30, as indicated by double arrow V, to make the thermoplastic fibres of the backing layer 28 to melt across the combined thickness of the two layers of wound dressing laminate 2a,2b in a pattern suited for delimiting the corresponding intended body-shape wound dressing 1. So the ultrasonic welding can create a welding at any desired position and at any size, including the welding seam 3 of the glove 1. The welded area, zone or seam has a certain minimum width, or can include the entire area of the two opposite layers of wound dressing laminate 2a,2b. Welding is excepted at areas that eventually become the compartment 6 of the body-shape wound dressing, the glove 1. After welding a continuous intermediate double wound dressing laminate product 32 is obtained, which continuous intermediate double wound dressing laminate product 32 has non-welded areas that delimit pockets that becomes the compartment 6 of the body-shape-wound dressing 1, and welded areas that later are part of the welding seam 3 of the body-shape wound dressing 1.

The continuous intermediate double wound dressing laminate product 32 is continuously cut into the shape of the body-shape wound dressings 1 at cutting station 33, as indicated by double arrow C, that either cut in welded areas to obtain the welding seam 3, or in pocket areas to obtain opening(s) 4. Waste wound dressing laminate 34 that does not form part of the body-shape wound dressing 1 is wound on a waste bobbin 35.

In the alternative cutting is also performed at the welding station.

### Examples of fibre layers

Data of some properties of suitable fibers and non-woven fibre layers before being made into the coherent wound laminate according to the present invention are given in the below Tables.

It should be noted that the Standards referred to in the Tables are different and therefore some data cannot be immediately compared. However the units given next to the data provide an indication of the selected property irrespective of the Standards.

Examples of the properties of a suitable felt of carboxymethyl cellulose fibres made into an absorbent material are given in Tables 1 and 2.

The fibres were sodium carboxymethyl cellulose fibres. The felt was made by first carding of carboxymethyl cellulose fibre tow, cross-lapping into the desired thickness, and then needle-punching to form integral felt. Tests were conducted by the applicant, and emphasis is made that thickness can be in the range of e.g. 0.5 mm - 5 mm for other sodium carboxymethyl cellulose fibre layers. The data of Table 1 and 2 are simply examples.

**Table 1**

| **TESTS PERFORMED** | **TEST STANDARD** | **SPECIFICATIONS** |
|---|---|---|
| Weight | ISO 536 | 100 g/m²±10% |
| Thickness | Pharmaplast | 1±0.2 mm |
| Absorption capacity of absorbent | EDANA10.1-72 | ≥ 20 g/cm² |
| Sealing strength of absorbent pad | ASTM F88 | >1.2 N/15mm |
| Sealing Itegrity of absorbent pad | ASTM 1929 | No leakage |
| Sterility test | BS EN 11737 | No growth |

**Table 2**

| **TESTS PERFORMED** | **TEST STANDARD** | **SPECIFICATIONS** |
|---|---|---|
| Weight | ISO 536 | 200g/m²±10% |
| Thickness | Pharmaplast | 1.9±0.2 mm |
| Absorption capacity of absorbent pad | EDANA10.1-72 | ≥ 30 g/cm² |
| Sealing strength of absorbent pad | ASTM F88 | >1.2 N/15mm |
| Sealing Integrity of absorbent pad | ASTM 1929 | No leakage |
| Sterility test | BS EN 11737 | No growth |

Table 3 shows data of a thermoplastic polyurethane fiber backing layer. Tests were made at Innovatec Microfibre Technology GmbH & Co. KG, Germany, who also provided the data.

**Table 3**

| **TESTS PERFORMED** | **TEST STANDARD** | **SPECIFICATIONS** |
|---|---|---|
| Weight | DIN EN 12127 | 70 g/m²±10% |
| Thickness | DIN EN ISO 9073-2:1997-02 | 0.3±0.1 mm |
| Tensile strength (dry), Machine direction | DIN EN 29073 T3 | > 30 N/5cm |
| Tensile strength (dry), Crosswise direction | DIN EN 29073 T3 | > 25 N/5cm |
| Elongation (dry), Machine direction | DIN EN 29073 T3 | >200 % |
| Elongation (dry), Crosswise direction | DIN EN 29073 T3 | >200 % |
| Air permeability | DIN EN ISO 9237 | 700±100 L/m²/s |
| Hydro head | DIN EN 20811 | >225 mm |

Table 4 below shows data of a spunlaced non-woven fibre backing layer. The fibers are obtainable from the French company Ahlstrom, who also provided the data.

**Table 4**

| **TESTS PERFORMED** | **TEST STANDARD** | **SPECIFICATIONS** |
|---|---|---|
| Weight | EDANA 40.3-90 | 42,3 - 51,7 g/m² |
| Thickness | EDANA 30.5-99 | 0,38 - 0,52 mm |
| Density | EDANA 30.5-99 | 81,3 - 136 kg/m³ |
| Tensile strength, Machine direction | EDANA 20.2-89 | 119 - 223 N |
| Tensile strength, Crosswise direction | EDANA 20.2-89 | 21,5 - 45,5 N |
| Elongation at break, Machine direction | EDANA 20.2-89 | 21,5 - 44,5 % |
| Elongation at break, Crosswise direction | EDANA 20.2-89 | 120 - 224 % |
| Mod 10% MD | I.O. 90 | MIN. 45 |
| Mod 10% CD | I.O. 90 | MIN. 1,60 |
| Web quality | I.O. 80 | MIN. K3 |

Table 5 below shows data of a non-woven backing layer of 100% hydrophobic polypropylene fibres obtainable from Everfront Industrial Co. Ltd, P.O. Box 43-435, Taipai 106, Taiwan, R.O.C, who also provided the data.

**Table 5**

| **TESTS PERFORMED** | **TEST STANDARD** | **SPECIFICATIONS** |
|---|---|---|
| Weight | CNS-1479 | 17 g/m² |
| Thickness | CNS-1479 | 0.16 mm |
| Density | CNS-1479 | 0.106 g/m³ |
| Tensile strength (dry), Machine direction | CNS-12815 | 3.3 kg |
| Tensile strength (dry), Crosswise direction | CNS-12815 | 2.5 kg |
| Elongation (dry), Machine direction | CNS-12815 | 75 % |
| Elongation (dry), Crosswise direction | CNS-12815 | 125 % |

The present invention unites the unique properties of the individual fibres and wound dressing laminate layers to create improved wound dressing laminates and improved body-shape wound dressings.

## Claims

1. A body-shape wound dressing comprising a wound dressing laminate (2,2a,2b), which wound dressing laminate comprises a wound-facing non-woven layer (7a,7b) composed of non-thermoplastic fibres (8) combined into a coherent laminate structure with a non-woven backing layer (9a,9b) composed of thermoplastic fibres (10), **characterised in that**
- the non-thermoplastic fibres (8) of the wound-facing non-woven layer (7a,7b) are carboxymethyl cellulose fibres,
- the thermoplastic fibres (10) of the non-woven backing layer (9a,9b) are selected from the group of fibres comprising polyurethane fibres, hydrophobic polypropylene fibres, and/or spunlaced fibres, wherein
- the wound-facing non-woven layer (7a,7b) and the non-woven backing layer (9a,9b) have been laminated together by needle-punching.

2. A body-shape wound dressing according to claim 1, **characterised in that** the wound-facing non-woven layer (7a, 7b) is a needle-punched felt (20).

3. A body-shape wound dressing according to claims 1 or 2, **characterised in that** the weight of the wound-facing non-woven layer (7a,7b) is between 50 g/m² and 250 g/m².

4. A body-shape wound dressing according to any of claims 1, 2 or 3, **characterised in that** the carboxymethyl cellulose fibres (8) comprises sodium carboxymethyl cellulose fibres.

5. A body-shape wound dressing according to any of the preceding claims 1 - 4, **characterised in that** the spunlaced fibres are spunlaced polyester fibres.

6. A body-shape wound dressing according to any of the preceding claims 1 - 5, **characterised in that** the wound dressing laminate (2,2a,2b) is manufactured without additional use of adhesives for coherency between any of the fibre layers (7a, 7b; 9a, 9b).

7. A body-shape wound dressing according to any of the preceding claims 1 - 6, **characterised in that** an antimicrobial agent is included in the wound-facing non-woven layer (7a,7b).

8. A body-shape wound dressing according to claim 7, **characterised in that** the antimicrobial agent includes one or more of ionic silver, silver aluminosilicate, glass silver or nano-silver.

9. A body-shape wound dressing according to any of the preceding claims 1 - 8, **characterised in that** the majority of fibres exposed at the non-thermoplastic felt side of the wound dressing laminate (2,2a,2b) are carboxymethyl cellulose fibres (8).

10. A body-shape wound dressing according to any of the preceding claims 1 - 9, **characterised in that** the majority of fibres exposed on the thermoplastic layer side are thermoplastic polyurethane fibres, hydrophobic polypropylene fibres and/or spunlaced fibres (10).

11. A body-shape wound dressing according to any of claims 1 or 10, **characterised in that** the body-shape wound dressing (1) is composed of at least two opposite wound dressing laminate layers (2a,2b) welded together along at least a part of adjacent edges by a welding seam (3).

12. A body-shape wound dressing according to claim 11, **characterised in that** the opposite wound dressing laminate layers (2a,2b) are welded together by ultrasonic welding.

13. A body-shape wound dressing according to any of the preceding claims 1 - 12, **characterised in that** the body-shape wound dressing (1) is configured as a tube, a glove, a T-shirt, a finger wrap, a glove, a mitt, a sleeve, a vest, a jacket, a mask, a skull cap, a neck tube, a girdle, shorts, pants, leggings, a leg wrap, a sock or a toe wrap.

14. A body-shape wound dressing according to any of the preceding claims 1 - 13, **characterised in that** a non-thermoplastic felt side of the wound dressing laminate (2,2a,2b) is the wound contacting side.

15. A method of manufacturing a body-shape wound dressing according to any of the claims 1 - 14, **characterised in that** the method comprises the steps of
- providing a non-woven fibre layer (7a,7b) composed of non-thermoplastic carboxymethyl cellulose fibres (8),
- providing, on top of the non-woven fibre layer (7a,7b) composed of non-thermoplastic carboxymethyl cellulose fibres (8), a non-woven fibre layer (9a,9b) composed of thermoplastic fibres (10) selected from the group of fibres (10) comprising polyurethane fibres, hydrophobic polypropylene fibres and/or spunlaced fibres,
- combining the non-woven fibre layer (7a,7b) composed of non-thermoplastic carboxymethyl cellulose fibres (8) and the non-woven fibre layer composed of thermoplastic fibres (10) selected from the group of fibres comprising polyurethane fibres, hydrophobic polypropylene fibres and/or spunlaced fibres into a coherent wound dressing laminate (2,2a,2b) by needle-punching, and
either
- cutting the pieces (2,2a,2b) for the body-shape wound dressing (1) from the wound dressing laminate (2,2a,2b), and welding together the pieces (2,2a,2b) at least partly along opposite cut edges of said pieces (2,2a,2b) thereby creating selected openings (4,5a,5b) for application of the body-shape wound dressing on a body part, or
- placing two wound dressing laminates (2,2a,2b) on top of each other, welding the two wound dressing laminates (2,2a,2b) to each other along a welding seam pattern (3) defining a selected body-shape wound dressing (1), with or without one or more openings (4,5a,5b) for application of the body-shape wound dressing (1) on a body part, and separating the welded body-shape wound dressing (1) from the opposite two wound dressing laminates (2,2a,2b) by cutting.

16. A method of manufacturing a body-shape wound dressing (1) according to claim 15, **characterised in that** the cutting step is die-cutting.

17. A method of manufacturing a body-shape wound dressing (1) according to any of the claims 15 or 16, **characterised in that** the welding step is ultrasonic welding.

18. A method of manufacturing a wound dressing laminate (2,2a,2b) as defined in any of the preceding claims 1 - 14 for use in a body-shape wound dressing according to any of the preceding claims 1 - 14, **characterised in that** the method comprises the steps of
- air-laying a non-woven fibre web composed of non-thermoplastic carboxymethyl cellulose fibres (8),
- air-laying a non-woven fibre web composed of thermoplastic fibres selected from the group of fibres (10) comprising polyurethane fibres, hydrophobic polypropylene fibres and/or spunlaced fibres on top of the non-woven fibre web composed of non-thermoplastic carboxymethyl cellulose fibres (8), and
- combining the non-woven fibre web composed of non-thermoplastic carboxymethyl cellulose fibres (8) and the non-woven fibre web composed of non-thermoplastic fibres (10) selected from the group of fibres comprising polyurethane fibres, hydrophobic polypropylene fibres and/or spunlaced fibres into a coherent laminate structure by needle-punching.

## Patentansprüche

1. Ein körperförmiger Wundverband umfassend ein Wundverbandlaminat (2, 2a, 2b), wobei das Wundverbandlaminat eine der wundzugewandte Vliesschicht (7a, 7b) aus nicht-thermoplastischen Fasern (8) umfasst, die zu einer kohärenten Laminatstruktur mit einer Vliesträgerschicht (9a, 9b) aus thermoplastischen Fasern (10) kombiniert sind, **dadurch gekennzeichnet, dass**
- die nicht-thermoplastischen Fasern (8) der der wundzugewandten Vliesschicht (7a, 7b) Carboxymethylcellulosefasern sind,
- die thermoplastischen Fasern (10) der Vliesträgerschicht (9a, 9b) aus der Gruppe von Fasern ausgewählt sind, die Polyurethanfasern, hydrophobe Polypropylenfasern und/oder gesponnenen Fasern umfassen,
- wobei die wundzugewandte Vliesschicht (7a,7b) und die Vliesträgerschicht (9a,9b) durch Nadel-Stanzen zusammen laminiert worden sind.

2. Ein körperförmiger Wundverband nach Anspruch 1, **dadurch gekennzeichnet, dass** die wundzugewandte Vliesschicht (7a, 7b) ein nadelgestanzter Filz (20) ist.

3. Ein körperförmiger Wundverband nach den Ansprüchen 1 oder 2, **dadurch gekennzeichnet, dass** das Gewicht der wundzugewandten Vliesschicht (7a, 7b) zwischen 50 g/m2 und 250 g/m2 beträgt.

4. Ein körperförmiger Wundverband nach einem der Ansprüche 1, 2 oder 3, **dadurch gekennzeichnet, dass** die Carboxymethylcellulosefasern (8) Natriumcarboxymethylcellulosefasern umfassen.

5. Ein körperförmiger Wundverband nach einem der vorhergehenden Ansprüche 1-4, **dadurch gekennzeichnet, dass** die gesponnenen Fasern gesponnene Polyesterfasern sind.

6. Ein körperförmiger Wundverband nach einem der vorhergehenden Ansprüche 1-5, **dadurch gekennzeichnet, dass** das Wundverbandlaminat (2, 2a, 2b) ohne zusätzliche Verwendung von Klebstoffen für die Kohärenz zwischen einer der Faserschichten (7a, 7b; 9a, 9b) hergestellt wird.

7. Ein körperförmiger Wundverband nach einem der vorhergehenden Ansprüche 1-6, **dadurch gekennzeichnet, dass** ein antimikrobielles Mittel in der wundzugewandten Vliesschicht (7a,7b) enthalten ist.

8. Ein körperförmiger Wundverband nach Anspruch 7, **dadurch gekennzeichnet, dass** das antimikrobielle Mittel eines oder mehrere von ionischem Silber, Silber-Aluminiumsilikat, Glas-Silber oder Nano-Silber enthält.

9. Ein körperförmiger Wundverband nach einem der vorhergehenden Ansprüche 1 bis 8, **dadurch gekennzeichnet, dass** die Mehrzahl der an der nicht-thermoplastischen Filzseite des Wundverbandlaminats (2, 2a, 2b) freiliegenden Fasern Carboxymethylcellulosefasern (8) sind.

10. Ein körperförmiger Wundverband nach einem der vorstehenden Ansprüche 1-9, **dadurch gekennzeichnet, dass** die Mehrzahl der auf der Seite der thermoplastischen Schicht freiliegenden Fasern thermoplastische Polyurethanfasern, hydrophobe Polypropylenfasern und/oder gesponnene Fasern sind (10).

11. Ein körperförmiger Wundverband nach einem der Ansprüche 1 oder 10, **dadurch gekennzeichnet, dass** der körperförmige Wundverband (1) aus mindestens zwei gegenüberliegenden Wundverbandlaminatschichten (2a, 2b) besteht, die entlang mindestens eines Teils der benachbarten Ränder durch eine Schweißnaht (3) miteinander verschweißt sind.

12. Ein körperförmiger Wundverband nach Anspruch 11, **dadurch gekennzeichnet, dass** die gegenüberliegenden Wundverbandlaminatschichten (2a, 2b) durch Ultraschallschweißen miteinander verschweißt sind.

13. Ein körperförmiger Wundverband nach einem der vorhergehenden Ansprüche 1 bis 12, **dadurch gekennzeichnet, dass** der körperförmige Wundverband (1) als ein Schlauch, ein Handschuh, ein T-Shirt, ein Fingerwickel, ein Handschuh, ein Halbhandschuh, ein Ärmel, eine Weste, eine Jacke, eine Maske, eine Schädelkappe, ein Halsschlauch, ein Gürtel, Shorts, Hose, Leggings, Beinwickel, eine Socke oder ein Zehenwickel konfiguriert ist.

14. Ein körperförmiger Wundverband nach einem der vorstehenden Ansprüche 1 - 13, **dadurch gekennzeichnet, dass** eine nicht-thermoplastische Filzseite des Wundverbandlaminats (2, 2a, 2b) die wundberührende Seite ist.

15. Ein Verfahren zur Herstellung eines körperförmigen Wundverbandes nach einem der Ansprüche 1 - 14, **dadurch gekennzeichnet, dass** das Verfahren die Schritte umfasst
- Bereitstellen einer Vliesschicht (7a,7b), die aus nicht-thermoplastischen Carboxymethylcellulosefasern (8) besteht,
- Bereitstellen einer Vliesfaserschicht (9a, 9b) aus thermoplastischen Fasern (10) ausgewählt aus der Gruppe von Fasern umfassend Polyurethanfasern, hydrophoben Polypropylenfasern
- und/oder gesponnenen Fasern auf der Vliesschicht (7a, 7b) aus nicht-thermoplastischen Carboxymethylcellulosefasern (8) auf der Faservlieslage (7a, 7b) aus nichtthermoplastischen Carboxymethylcellulosefasern (8),
- Kombinieren der Vliesschicht (7a, 7b) aus nicht-thermoplastischen Carboxymethylcellulosefasern (8) und der Vliesschicht aus thermoplastischen Fasern (10) ausgewählt aus der Gruppe der Fasern umfassend Polyurethanfasern, hydrophoben Polypropylenfasern und/oder gesponnene Fasern zu einem zusammenhängenden Wundverbandlaminat (2,2a, 2b) durch Nadel-Stanzen, und
entweder
- Schneiden der Teile (2,2a, 2b) für den körperförmigen Wundverband (1) aus dem Wundverbandlaminat (2,2a, 2b) und Zusammenschweißen der Teile (2,2a, 2b) zumindest teilweise entlang der gegenüberliegenden Schnittkanten der genannten Teile (2,2a, 2b), wodurch ausgewählte Öffnungen (4,5a, 5b) zum Aufbringen des körperförmigen Wundverbandes auf ein Körperteil erzeugt werden, oder
- Platzieren der zwei Wundverbandlaminate (2,2a, 2b) übereinander, Verschweißen der zwei Wundverbandlaminate (2,2a, 2b) miteinander entlang eines Schweißnahtmusters (3), das einen ausgewählten körperförmigen Wundverband definiert (1) mit oder ohne eine oder mehrere Öffnungen (4,5a, 5b) zum Aufbringen des körperförmigen Wundverbandes (1) auf ein Körperteil, und Trennen des geschweißten körperförmigen Wundverbandes (1) von den zwei gegenüberliegenden Wundverbandlaminaten (2,2a, 2b) durch Schneiden.

16. Ein Verfahren zur Herstellung eines körperförmigen Wundverbandes (1) nach Anspruch 15, **dadurch gekennzeichnet, dass** der Schneideschritt ein Stanzen ist.

17. Ein Verfahren zur Herstellung eines körperförmigen Wundverbandes (1) nach einem der Ansprüche 15 oder 16, **dadurch gekennzeichnet, dass** der Schweißschritt Ultraschallschweißen ist.

18. Ein Verfahren zur Herstellung eines körperförmigen Wundverbandes (2, 2a, 2b) nach einem der vorhergehenden Ansprüche 1 bis 14 zur Verwendung in einem körperförmigen Wundverband nach einem der vorhergehenden Ansprüche 1 bis 14, **dadurch gekennzeichnet, dass** das Verfahren die Schritte umfasst
- Luftlegen einer Faservliesbahn aus nicht-thermoplastischen Carboxymethylcellulosefasern (8),
- Auflegen einer Faservliesbahn aus thermoplastischen Fasern ausgewählt aus der Gruppe von Fasern (10) umfassend Polyurethanfasern, hydrophobe Polypropylenfasern und/oder gesponnenen Fasern, auf die Faservliesbahn aus nicht thermoplastischer Carboxymethylcellulose Fasern (8) und
- Kombinieren der Faservliesbahn aus nicht-thermoplastischen Carboxymethylcellulosefasern (8) und der Faservliesbahn aus nicht-thermoplastischen Fasern (10) ausgewählt aus der Gruppe der Fasern umfassend Polyurethanfasern, hydrophoben Polypropylenfasern und/oder gesponnenen Fasern durch Nadel-Stanzen zu einer zusammenhängenden Laminatstruktur.

## Revendications

1. Pansement qui épouse la configuration du corps comprenant un stratifié qui fait office de pansement (2, 2a, 2b), ledit stratifié qui fait office de pansement comprenant une couche sous la forme d'un non-tissé (7a, 7b) orientée en direction de la blessure, qui se compose de fibres non thermoplastiques (8), combinée, de façon à obtenir une structure de stratifié cohérente, avec une couche de support sous la forme d'un non-tissé (9a, 9b), qui se compose de fibres thermoplastiques (10), **caractérisé en ce que**
- les fibres non thermoplastiques (8) de la couche sous la forme d'un non-tissé (7a, 7b) orientée en direction de la blessure représentent des fibres de carboxyméthylcellulose ;
- les fibres thermoplastiques (10) de la couche de support sous la forme d'un non-tissé (9a, 9b) sont choisies parmi le groupe de fibres comprenant des fibres de polyuréthane, des fibres hydrophobes de polypropylène et/ou des fibres lacées par filage ; dans lequel
- la couche sous la forme d'un non-tissé (7a, 7b) orientée en direction de la blessure et la couche de support sous la forme d'un non-tissé (9a, 9b) ont été stratifiées l'une à l'autre par aiguilletage.

2. Pansement qui épouse la configuration du corps selon la revendication 1, **caractérisé en ce que** la couche sous la forme d'un non-tissé (7a, 7b) orientée en direction de la blessure est un feutre à l'aiguille (20).

3. Pansement qui épouse la configuration du corps selon la revendication 1 ou 2, **caractérisé en ce que** le poids de la couche sous la forme d'un non-tissé (7a, 7b) orientée en direction de la blessure se situe entre 50 g/m² et 250 g/m².

4. Pansement qui épouse la configuration du corps selon l'une quelconque des revendications 1, 2 ou 3, **caractérisé en ce que** les fibres de carboxyméthylcellulose (8) comprennent des fibres de carboxyméthylcellulose de sodium.

5. Pansement qui épouse la configuration du corps selon l'une quelconque des revendications précédentes 1 à 4, **caractérisé en ce que** les fibres lacées par filage représentent des fibres de polyester lacées par filage.

6. Pansement qui épouse la configuration du corps selon l'une quelconque des revendications précédentes 1 à 5, **caractérisé en ce que** le stratifié qui fait office de pansement (2, 2a, 2b) est fabriqué en l'absence d'une utilisation supplémentaire d'adhésifs pour obtenir une cohérence entre les couches de fibres (7a, 7b ; 9a, 9b) quelles qu'elles soient.

7. Pansement qui épouse la configuration du corps selon l'une quelconque des revendications précédentes 1 à 6, **caractérisé en ce qu'**un agent antimicrobien est inclus dans la couche sous la forme d'un non-tissé (7a, 7b) orientée en direction de la blessure.

8. Pansement qui épouse la configuration du corps selon la revendication 7, **caractérisé en ce que** l'agent antimicrobien englobe un ou plusieurs agents choisis parmi de l'argent ionique, de l'aluminosilicate d'argent, du verre argenté ou du nano-argent.

9. Pansement qui épouse la configuration du corps selon l'une quelconque des revendications précédentes 1 à 8, **caractérisé en ce que** la majeure partie des fibres exposées du côté feutre non thermoplastique du stratifié qui fait office de pansement (2, 2a, 2b) représentent des fibres de carboxyméthylcellulose (8).

10. Pansement qui épouse la configuration du corps selon l'une quelconque des revendications précédentes 1 à 9, **caractérisé en ce que** la majeure partie des fibres exposées du côté couche thermoplastique représente des fibres de polyuréthane thermoplastique, des fibres hydrophobes de polypropylène et/ou des fibres lacées par filage (10).

11. Pansement qui épouse la configuration du corps selon l'une quelconque des revendications 1 ou 10, **caractérisé en ce que** le pansement qui épouse la configuration du corps (1) se compose d'au moins deux couches opposées (2a, 2b) sous la forme d'un stratifié qui fait office de pansement, soudées l'une à l'autre le long d'au moins une partie de bords adjacents par l'intermédiaire d'un cordon de soudure (3).

12. Pansement qui épouse la configuration du corps selon la revendication 11, **caractérisé en ce que** les couches opposées (2a, 2b) sous la forme d'un stratifié qui fait office de pansement sont soudées l'une à l'autre par l'intermédiaire d'un soudage à ultrasons.

13. Pansement qui épouse la configuration du corps selon l'une quelconque des revendications précédentes 1 à 12, **caractérisé en ce que** le pansement qui épouse la configuration du corps (1) est configuré sous la forme d'un tube, d'un gant, d'un T-shirt, d'un élément enveloppant destiné à un doigt, d'un gant, d'une mitaine, d'une manche, d'une veste, d'un blouson, d'un masque, d'une calotte, d'un tube pour le cou, d'une gaine, d'un short, d'un pantalon, de collants, d'un élément enveloppant destiné à une jambe, d'une chaussette ou d'un élément enveloppant destiné à un orteil.

14. Pansement qui épouse la configuration du corps selon l'une quelconque des revendications précédentes 1 à 13, **caractérisé en ce qu'**un côté feutre non thermoplastique du stratifié qui fait office de pansement (2, 2a, 2b) représente le côté qui entre en contact avec la blessure.

15. Procédé de fabrication d'un pansement qui épouse la configuration du corps selon l'une quelconque des revendications 1 à 14, **caractérisé en ce que** le procédé comprend les étapes consistant à :
- procurer une couche fibreuse sous la forme d'un non-tissé (7a, 7b) qui se compose de fibres non thermoplastiques (8) à base de carboxyméthylcellulose ;
- procurer, par-dessus la couche fibreuse sous la forme d'un non-tissé (7a, 7b) qui se compose de fibres non thermoplastiques (8) à base de carboxyméthylcellulose, une couche fibreuse sous la forme d'un non-tissé (9a, 9b), qui se compose de fibres thermoplastiques (10) choisies parmi le groupe de fibres (10) comprenant des fibres de polyuréthane, des fibres hydrophobes de polypropylène et/ou des fibres lacées par filage ;
- combiner la couche fibreuse sous la forme d'un non-tissé (7a, 7b) qui se compose de fibres non thermoplastiques (8) à base de carboxyméthylcellulose et la couche fibreuse sous la forme d'un non-tissé (9a, 9b), qui se compose de fibres thermoplastiques (10) choisies parmi le groupe de fibres (10) comprenant des fibres de polyuréthane, des fibres hydrophobes de polypropylène et/ou des fibres lacées par filage afin d'obtenir un pansement cohérent sous la forme d'un stratifié (2, 2a, 2b) qui fait office de pansement par l'intermédiaire d'un aiguilletage ; et
soit
- découper les pièces (2, 2a, 2b) destinées au pansement (1) qui épouse la configuration du corps à partir du stratifié qui fait office de pansement (2, 2a, 2b) et souder ensemble les pièces (2, 2a, 2b) au moins en partie le long de bords de découpe opposés desdites pièces (2, 2a, 2b) pour ainsi créer des ouvertures (4, 5a, 5b) qui ont été sélectionnées pour l'application du pansement qui épouse la configuration du corps sur une partie du corps; soit
- placer deux stratifiés qui font office de pansements (2, 2a, 2b) l'un par-dessus l'autre ; souder les deux stratifiés qui font office de pansements (2, 2a, 2b) l'un à l'autre le long d'un motif d'un cordon de soudure (3) définissant un pansement sélectionné (1) qui épouse la configuration du corps, avec ou sans une ou plusieurs ouvertures (4, 5a, 5b) pour l'application du pansement qui épouse la configuration du corps (1) sur une partie du corps ; et séparer le pansement soudé qui épouse la configuration du corps (1) par rapport aux deux stratifiés qui font office de pansements (2, 2a, 2b) par découpe.

16. Procédé de fabrication d'un pansement (1) qui épouse la configuration du corps selon la revendication 15, **caractérisé en ce que** l'étape de découpe représente un découpage à l'emporte-pièce.

17. Procédé de fabrication d'un pansement (1) qui épouse la configuration du corps selon l'une quelconque des revendications 15 ou 16, **caractérisé en ce que** l'étape de soudage représente un soudage par ultrasons.

18. Procédé fabrication d'un stratifié qui fait office de pansement (2, 2a, 2b) tel que défini dans l'une quelconque des revendications précédentes 1 à 14 pour son utilisation dans un pansement qui épouse la configuration du corps selon l'une quelconque des revendications précédentes 1 à 14, **caractérisé en ce que** le procédé comprend les étapes consistant à :
- arranger par flux d'air une bande fibreuse sous la forme d'un non-tissé qui se compose de fibres non thermoplastiques (8) à base de carboxyméthylcellulose ;
- arranger par flux d'air une bande fibreuse sous la forme d'un non-tissé qui se compose de fibres thermoplastiques choisies parmi le groupe de fibres (10) comprenant des fibres de polyuréthane, des fibres hydrophobes de polypropylène et/ou des fibres lacées par filage par-dessus la bande fibreuse sous la forme d'un non-tissé qui se compose de fibres non thermoplastiques (8) à base de carboxyméthylcellulose ; et
- combiner la bande fibreuse sous la forme d'un non-tissé qui se compose de fibres non thermoplastiques (8) à base de carboxyméthylcellulose et la bande fibreuse sous la forme d'un non-tissé qui se compose de fibres thermoplastiques choisies parmi le groupe de fibres (10) comprenant des fibres de polyuréthane, des fibres hydrophobes de polypropylène et/ou des fibres lacées par filage de façon à obtenir une structure de stratifié cohérente par aiguilletage.
